# EUROPÄISCHE PATENTANMELDUNG

(11) **EP 0 617 929 A2**
(43) Veröffentlichungstag der Anmeldung: **05.10.1994**
(21) Anmeldenummer: 93119398.1
(22) Anmeldetag: 02.12.1993
(51) Int. Cl.: A61D 17/00

(54) **Verfahren und Vorrichtung zur Überwachung und Anzeige des Beginns einer Geburt bei Pferden**

(30) Priorität: 19.02.1993 DE 4305131
(71) Anmelder: Kegel, Werner, D-49536 Lienen (DE)
(72) Erfinder: Kegel, Werner, D-49536 Lienen (DE)
(74) Vertreter: Busse & Busse Patentanwälte

(57) **Zusammenfassung**

Die Erfindung bezieht sich auf ein Verfahren zur Überwachung und Anzeige des Beginns einer Geburt bei Pferden. Als Indikator für den Beginn der Geburt wird der schnelle zeitliche Anstieg der Schweißabsonderung des Muttertieres kurz vor der Geburt benutzt. Die Schweißabsonderung des Muttertieres wird von einem Meßfühler (2) überwacht. Anschließend wird das Meßsignal von einem Signalfilter (3) in der Weise verarbeitet, daß langzeitige Änderungen der Schweißabsonderung ausgefiltert werden und schnelle zeitliche Änderungen verstärkt werden. Eine Vorrichtung (1) zur Durchführung des Verfahrens sieht einen Meßfühler (2), einen Signalfilter (3), einen Grenzwertgeber (4) und eine Halteschaltung (5) vor.

## Beschreibung

Die Erfindung bezieht sich auf ein Verfahren zur Überwachung und Anzeige des Beginns einer Geburt bei Pferden nach dem Oberbegriff des Anspruchs 1 sowie eine Vorrichtung nach dem Oberbegriff des Anspruchs 6.

Eine in der DE-PS 27 19 698 beschriebene Vorrichtung soll den Züchter in die Lage versetzen, Kenntnis von dem Beginn der Geburt eines Fohlens zu erhalten, ohne selbst beim Muttertier anwesend zu sein. Es ist bekannt, als Meßgröße die vermehrte Schweißabsonderung von Stuten kurz vor der Geburt zu benutzen. Überschreitet die Schweißabsonderung des Muttertieres einen vorgegebenen Grenzwert, der individuell einstellbar ist, so gilt dies als Indikator für den Beginn der Geburt des Fohlens.

In der Praxis hat es sich jedoch gezeigt, daß die Schweißabsonderung der Muttertiere je nach Nervosität und Bewegungsdrang, je nach Wetterverhältnissen und Stallklima, je nach Dicke des Felles oder sonstiger Befindlichkeit des Tieres sehr unterschiedlich sein kann. Die Stärke der Schweißabsonderung eines Muttertieres ist deshalb oft als alleiniger Indikator für den Beginn einer Geburt bei Pferden nicht zuverlässig genug. Zur Berücksichtigung unterschiedlicher Einflußgrößen ist die vorbekannte Ausführungsform mit einer Einstellmöglichkeit für den Grenzwert zur Alarmsignalgabe ausgestattet. Dies schafft in jedem Fall die Notwendigkeit einer Einschätzung mit einem entsprechenden Fehlerrisiko. Darüber hinaus können Fehlereinstellungen durch Unkenntnis, Unachtsamkeit oder versehentliche Betätigungen vorkommen. Ein Grenzwert, wie er bei Vorrichtungen in Anlehnung an die DE-PS 27 19 698 einstellbar ist, schafft Ungewißheiten hinsichtlich der Warnsignalgabe, wenn nicht genau bekannt ist, inwieweit eine Schweißabsonderung aufgrund von Phänomenen, die ja gerade nicht angezeigt werden sollen, abweichen oder schwanken kann.

Aufgabe der Erfindung ist es, eine Überwachung und Anzeige der Fohlengeburt höherer Zuverlässigkeit bei einer einfachen Handhabung und hoher Bedienungssicherheit und Robustheit zu schaffen.

Gemäß der Erfindung wird diese Aufgabe von einem Verfahren nach dem Oberbegriff des Anspruchs 1 ausgehend durch die kennzeichnenden Merkmale des Anspruchs 1 sowie von einer Vorrichtung nach dem Oberbegriff des Anspruchs 6 mit den kennzeichnenden Merkmalen des Anspruchs 6 gelöst.

Das Verfahren nach der Erfindung sieht vor, die zeitlichen Änderungen in der Schweißabsonderung eines Pferdes zu erfassen. Da festgestellt wurde, daß es kurz vor der Geburt zu einer vermehrten, sehr schnell ansteigenden Schweißabsonderung kommt, ist es sinnvoll, diesen schnellen Anstieg der Schweißabsonderung meßtechnisch zu erfassen und die langzeitige, aufgrund anderweitiger Umstände bedingte Schweißabsonderung meßtechnisch zu unterdrücken. Entscheidend ist also nicht die Stärke der Schweißabsonderung, sondern deren zeitliche Änderung.

Damit wird ein für den Beginn der Geburt sehr viel signifikanteres Meßsignal gewonnen, welches eine höhere Zuverlässigkeit der Überwachung erzielt. Dies schafft eine wesentliche Sicherheit in der Überwachung und einen Schutz gegen Fehlanzeigen.

Die mit der Erfindung erzielten Vorteile bestehen auch darin, daß die Einstellung eines Sollwertes wegfällt. Irrtümer, Fehler oder Unachtsamkeiten in der Einstellung entfallen. Ein insgesamt hermetisch abschließbares Gerät läßt sich robust und stallgerecht ausführen, darüber hinaus preisgünstig herstellen, da der Aufwand für interne elektronische Bauteile meist weniger zu Buche schlägt als der Aufwand für externe Bedienelemente. Insbesondere wird die Bedienerfreundlichkeit erhöht, da sich der Züchter keine Gedanken über den jeweiligen situtationsbedingten Sollwert machen muß und eine fehlende oder falsche Anzeige des Beginns einer Geburt durch eine falsche Vorwahl des Sollwertes entfällt.

Zweckmäßig kann das Meßsignal zunächst durch einen Tiefpaßfilter geglättet werden, um Störsignale mit Schwankungen im Sekundenbereich auszufiltern, die durch Bewegungen des Pferdes, Verschiebungen des Meßfühlers gegenüber dem Fell, unregelmäßige Schweißabsonderungen oder dergleichen auftreten können. In jedem Fall ist sicherzustellen, daß elektronische Einstreuungen ohne Einfluß bleiben.

Vorteilhafterweise wird das einmal vom Grenzwertgeber festgestellte Geburtsbeginn-Signal dauernd oder zumindest über einen vorgegebenen Zeitraum gehalten, damit eine ausreichende Alarmdauer gewährleistet ist.

Die erfindungsgemäße Vorrichtung kann sich zur Erkennung von Meßsignaländerungen zweckmäßig eines verzweigten Signalfilters bedienen, bei dem ein Filterzweig mit einem Tiefpaßfilter versehen ist, dessen Ausgangssignal von einem den zweiten Filterzweig durchlaufenden Signal abgezogen wird. Im Ergebnis führt dies zu einem Hochpaßfilter ohne die für Störsignale anfälligere Konfiguration von Differentiations-Schaltungen oder Kondensator-Ketten zu verwenden.

Zur weiteren Erläuterung der Erfindung wird auf die Zeichnung und die nachfolgende Beschreibung verwiesen. In der Zeichnung zeigen:
- **Fig. 1**: ein Blockschaltbild der Vorrichtung,
- **Fig. 2**: zeigt ein Schaltschema eines Blocks aus Fig. 1,
- **Fig. 3**: zeigt Signalverläufe in der Vorrichtung.

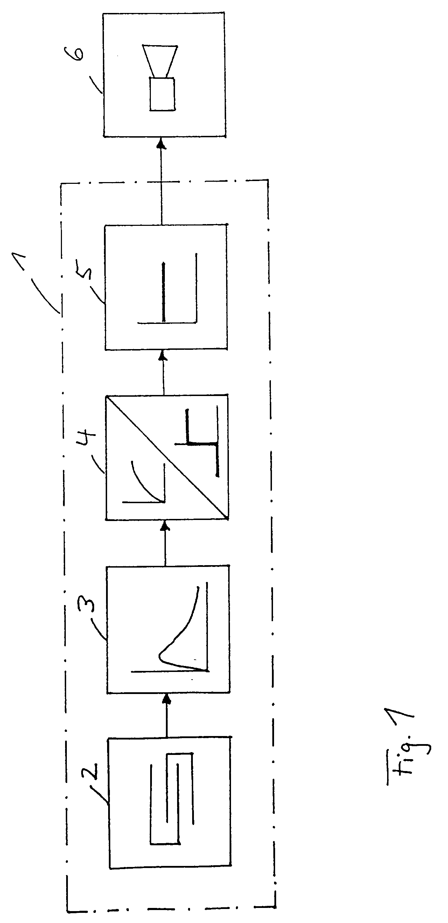

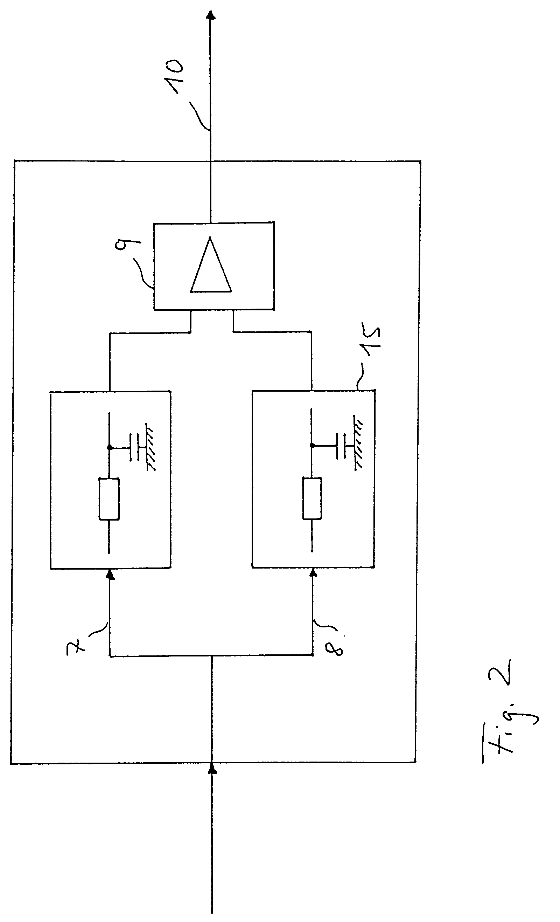

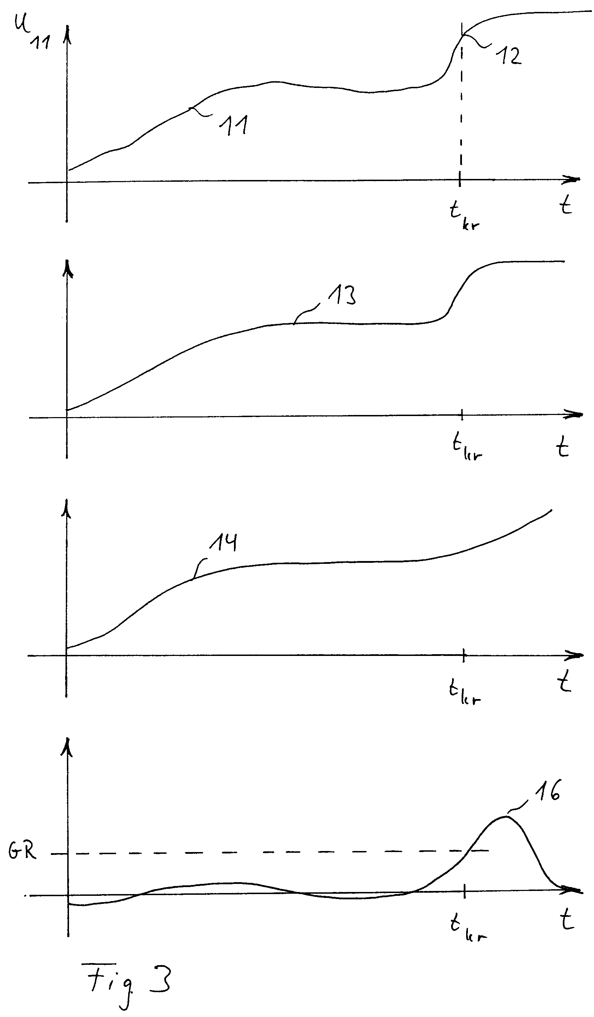

Fig. 1 zeigt die Vorrichtung 1 zur Überwachung einer Geburt bei Pferden im Blockschaltbild. Die Vorrichtung besteht aus einem Meßfühler 2, der seinen elektrischen Widerstand in Abhängigkeit von der Schweißfeuchtigkeit eines Muttertieres ändert, einem Signalfilter 3, einem Grenzwertgeber 4, einer Halteschaltung 5 und einem Alarm- und Anzeigegerät 6. Das vom Meßfühler kommende Meßsignal wird im Signalfilter 3 in einer unten anhand der Fig. 2 und 3 noch zu erläuternden Weise verarbeitet. Erreicht das verarbeitete Meßsignal einen bestimmten Grenzwert, so löst der Grenzwertgeber 4 eine Warnsignalgabe im Anzeigegerät 6 aus. Die Halteschaltung 5 gewährleistet, daß die Warnsignale über eine bestimmte zeitliche Mindestdauer fortbestehen. Das in Fig. 1 eingezeichnete Anzeigegerät 6 kann in vorbekannter Art als optischer und/oder akustischer Signalgeber ausgebildet sein. Vorzugsweise gelangt das Ausgangssignal der Halteschaltung 5 "drahtlos", also über eine Funkstrecke mit Sender und Empfänger vom Muttertier zu Aufenthalts-, Wohn- oder Schlafbereichen des Züchters. Dies hat gegenüber einer Kabelübertragung den Vorteil uneingeschränkter und ungestörter Bewegungsfreiheit des Muttertiers und einer nicht drahtgebundenen Verwendung der Vorrichtung. Ein dazu vorgesehener batteriebetriebener Sender wird beispielsweise nur in vorgegebenen Intervallen von z.B. 30 Sec. kurzzeitig aktiviert und überträgt ein Signal, wenn der Grenzwertgeber zwischenzeitlich angesprochen hat. Die Halteschaltung 5 muß dazu eine Mindestdauer-Haltezeit von dem vorgenannten Intervall (z.B. 30 Sec.) aufweisen.

Fig. 2 stellt eine zweckmäßige Ausführungsform des Signalfilters 3 dar. Das vom Meßfühler 2 kommende, verstärkte Meßsignal wird im Signalfilter 3 auf zwei Signalzweige 7 und 8 geleitet. Das Meßsignal in Signalzweig 7 durchläuft einen Tiefpaß, der Frequenzanteile mit Frequenzen größer als 0,2 Hz unterdrückt. Das Meßsignal in Signalzweig 8 durchläuft einen Tiefpaß, der Frequenzen ab 0,01 Hz unterdrückt. Von beiden in dieser Weise gebildeten Signalen wird in einem Summierer 9 ein Differenzsignal gebildet, das dann über eine Leitung 10 zum Grenzwertgeber geleitet wird.

Fig. 3 veranschaulicht die Signalverläufe nach der Vorrichtung. Hierbei ist ein Signal U11 am Eingang des Signalfilters 3 als Maß für die Schweißbefeuchtung des Meßfühlers 2 aufgetragen. Der Meßfühler 2 weist hierzu eine (nicht dargestellte) Meßplatte mit Elektroden auf, zwischen denen eine durch Schweißfeuchtigkeit überbrückbare Isolierstrecke einen Befeuchtungsgrad zu messen erlaubt. Dieses Signal wird im Meßfühler 2 verstärkt, wobei die sich an den Meßfühler 2 anschließenden Signalfilter 3, Grenzwertmelder 4 und Halteschaltung 5 in einem kompakten, mit einem Geschirr am Bug des Muttertieres angebrachten Gehäuses zusammengefaßt sind. Einen beispielhaften Meßsignalverlauf, der auch eine Geburtsbeginn-Phase 12 umfaßt, zeigt 11. In diese Phase 12 fällt ein Zeitpunkt tₖᵣ, zu dem ein Warnsignal ausgelöst werden soll. Das im Signalzweig 7 verarbeitete Signal, das lediglich durch Unterdrückung höherer Frequenzen geglättet ist, stellt ein Signalverlauf 13 dar. Mit 14 ist ein Signal bezeichnet, daß den Signalzweig 8 durchlaufen hat. Die Einstellung eines Tiefpasses 15 im Signalzweig 8 bewirkt eine deutlich weitergehende Glättung des Signals. Die Differenz beider Signale 13 und 14 wird in einem Signalverlauf 16 veranschaulicht. Die Abbildung zeigt, daß ein Grenzwert GR gesetzt werden kann, der ein verläßlicher Indikator für den Beginn der Geburt des Fohlens ist.

Das Verfahren ist dadurch gekennzeichnet, daß die zeitliche Änderung des Meßsignals zur Anzeige des Beginns einer Geburt bei Pferde verwendet wird. Nicht die absolute Schweißabsonderung des Pferdes als Indikator für den Eintritt in die Phase der Geburt des Fohlens, sondern die schnelle zeitliche Änderung der Schweißabsonderung des Muttertieres im Zeitbereich von bis zu 1 Minute Anstiegsdauer wird mit der beschriebenen Vorrichtung detektiert. Wird ein bestimmter Grenzwert der zeitlichen Änderung des Meßsignalverlaufs überschritten, so löst der Grenzwertgeber 4 eine Warnsignalgabe im Anzeigegerät 6 aus. Da nach dem schnellen Anstieg der Schweißabsonderung sich diese in der Regel auf einem hohen Niveau stabilisiert, wird der Grenzwert nach einer gewissen Zeitspanne, die sich in ihrer zeitlichen Länge im Rahmen der Anstiegsdauer der Erhöhung der Schweißabsonderung bewegt, wieder unterschritten. Damit jedoch weiterhin die Warnsignalgabe aufrechterhalten bleibt, ist zwischen dem Grenzwertgeber 4 und dem Alarmsignalgerät 6 eine Halteschaltung 5 eingebaut, die das Warnsignal auch nach Unterschreiten des Grenzwertes weiterhin für eine vorbestimmte Zeitdauer oder dauernd (bis zu einer Abschaltung von Hand) aufrechterhält.

Eine weitere Ausführungsform der vorliegenden Erfindung besteht darin, die zeitliche Entwicklung der Meßwerte von einem mit dem Meßwertaufnehmer verbundenen Mikroprozessor vornehmen zu lassen. Dies setzt zwar eine Abtastung und eine Analog-Digital-Umwandlung vor der zahlenmäßigen Verarbeitung im Mikroprozessor voraus, gibt aber die großen Freiheiten in der Einrichtung der Rechenalgorithmen, die für die digitale Verarbeitung typisch sind. Im einfachsten Fall werden dem Mikroprozessor Differenzengleichungen vorgegeben, die der Fachmann durch Umrechnung der analogen Filterfunktionen erhält.

## Patentansprüche

1. Verfahren zur Überwachung und Anzeige des Beginns einer Geburt bei Pferden mit Hilfe einer Meßsignal-Aufnahme von einem Meßfühler, der am Fell des Muttertieres angelegt ist und die Schweißfeuchtigkeit des Muttertieres überwacht und einer Übertragung des Meßsignals zu einem Grenzwertgeber, der bei Erreichen ein Anzeigegerät zur optischen und/oder akustischen Warnsignalgabe ansteuert,
dadurch gekennzeichnet,
daß das Meßsignal in der Weise verarbeitet wird, daß zeitliche Änderungen des elektrischen Meßsignals verstärkt und langzeitig konstante Signalanteile abgeschwächt werden und daß das derart verarbeitete Meßsignal in den Grenzwertgeber eingegeben wird.

2. Verfahren nach Anspruch 1,
dadurch gekennzeichnet,
daß das vom Meßfühler (2) kommende elektrische Meßsignal einen Tiefpaß zur Ausfilterung von höherfrequenten Signalanteilen mit Frequenzen von mehr als 0,2 Hz durchläuft.

3. Verfahren nach Anspruch 1 oder 2,
dadurch gekennzeichnet,
daß das geglättete Meßsignal einer Hochpaßfilterung unterzogen wird zur Unterdrückung von Frequenzen von weniger als 0,01 Hz.

4. Verfahren nach einem der Ansprüche 1 bis 3,
dadurch gekennzeichnet,
daß ein vom Grenzwertgeber ausgehendes Ansteuerungssignal drahtlos mit Hilfe eines Senders übertragen wird.

5. Verfahren nach einem der Ansprüche 1 bis 3,
dadurch gekennzeichnet,
daß ein vom Grenzwertgeber ausgehendes Ansteuerungssignal eines Warnanzeigegerätes zumindest über eine vorgegebene zeitliche Mindestdauer aufrechterhalten wird.

6. Vorrichtung zur Durchführung des Verfahrens nach einem der Ansprüche 1 bis 5,
mit einem am Fell des Muttertieres anzulegenden Meßfühler, einer ein vom Meßfühler ausgehendes Meßsignal auswertenden Schalteinrichtung mit einem Grenzwertgeber (4), die ein Anzeigegerät (6) zur optischen und/oder akustischen Warnsignalgabe ansteuert,
dadurch gekennzeichnet,
daß dem Grenzwertgeber im Anzeigegerät ein Signalfilter (3) vorgeschaltet ist, welches langzeitig konstante Signalanteile unterdrückt.

7. Vorrichtung nach Anspruch 6,
dadurch gekennzeichnet,
daß das Signalfilter (3) zwei Signalzweige (7,8) umfaßt, von denen ein Signalzweig einen Tiefpaßfilter umfaßt, der Frequenzanteile des Meßsignals von weniger als 0,01 Hz unterdrückt, und daß das Ausgangssignal des Tiefpaßfilters von einem den zweiten Signalzweig durchlaufenden Signal abgezogen wird.

8. Vorrichtung nach Anspuch 7,
dadurch gekennzeichnet,
daß in dem zweiten Signalzweig ein Tiefpaßfilter liegt, der Frequenzen des Meßsignals von mehr als 0,1 Hz unterdrückt.

9. Vorrichtung nach Anspruch 7 oder 8,
dadurch gekennzeichnet,
daß die Ausgangssignale der beiden Signalzweige in gegensinnige Eingange eines Verstärkers eingegeben werden.

10. Vorrichtung nach Anspruch 9,
dadurch gekennzeichnet,
daß dem Verstärker der Grenzwertgeber (4) direkt nachgeschaltet ist, dessen Ausgangssignal in eine Halteschaltung (5) einläuft.
